# EUROPEAN PATENT APPLICATION

(11) **EP 4 785 896 A1**
(43) Date of publication of application: **05.08.2026**
(21) Application number: 25155153.7
(22) Date of filing: 31.01.2025
(51) Int. Cl.: A61B 34/30, A61B 50/13

(54) **ROBOTIC SYSTEM FOR USE IN SURGERY, MICROSURGERY OR SUPER-MICROSURGERY**

(71) Applicant: Microsure B.V., 5692 EA Son (NL)
(72) Inventor: VAN DER WALLE, Dirk, 6861 BB, Oosterbeek (NL)
(74) Representative: WENDE IP GbR

(57) **Abstract**

The invention provides a robotic system (100) for use in surgery, microsurgery or super-microsurgery, comprising: a base unit (10); one or more robot arms (32), and a positioning and suspension system including a beam assembly and a lift mechanism (34). The beam assembly (24) comprises: a first beam (26); a second beam (28); and a C-shaped beam (30), configured to hold the one or more robot arms (32). The rotational axes of the first beam (26), the second beam (28), and the C-shaped beam (30) are parallel to each other. The lift mechanism (34) has a first end connected to the base unit (10) and a second end rotatably connected to the first beam (26), and is configured to move in the upward or downward direction along its translational axis, parallel with respect to the rotational axes of the first beam (26), the second beam (28), and the C-shaped beam (30).

## Description

The present invention belongs to the technical field of robotic systems for use in surgery, microsurgery or super-microsurgery.

In surgical procedures, in particular microsurgical or super-microsurgical procedures, robotic systems are used for precise surgical handlings on the submillimeter scale.

The use of robotic systems facilitates accurate movements with micrometer precision without substantial tremor, thereby superseding the limitations of human hand manipulation.

However, it is required that these robotic systems provide the operator (e.g., a surgeon) with sufficient dexterity, so as to be able to perform complex movements in the surgical, microsurgical or super-microsurgical workspace, further avoiding the risk of collision with other objects during manipulation.

In addition, it is important that parts of the robotic system, in particular the robot arm, do not interfere with, and thus obstruct, the microscopic view of the operator.

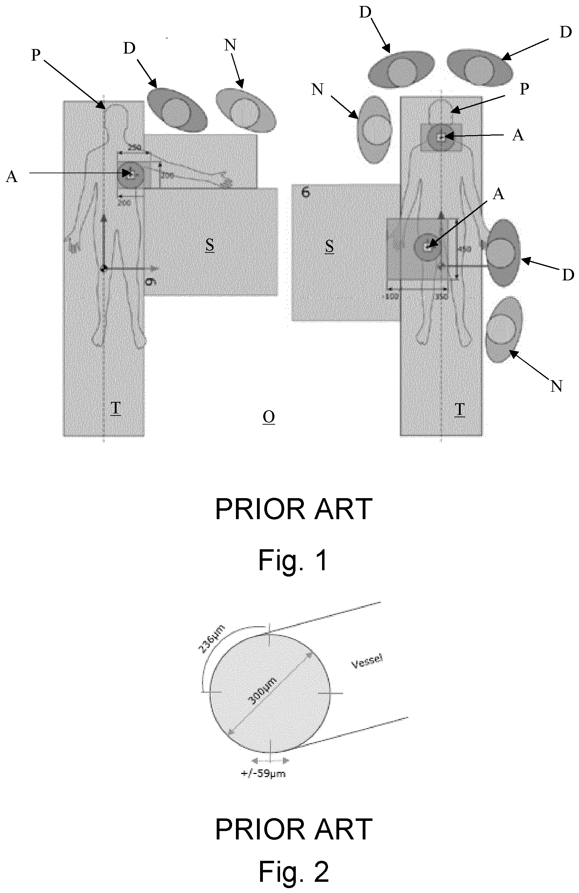

Fig. 1 provides a schematic view of an operating room O with a patient P lying on an operating table T, where areas of the patient P denoted with A represent surgical locations (wound areas) that must be reached with a robotic system S to perform a surgical, microsurgical or super-microsurgical procedure.

In particular, Fig. 1 provides two examples, respectively referring to a Lymphaticovenous anastomosis (LVA) procedure in the axilla (*left*) and a procedure involving free flap surgery in the neck and in the lower leg (*right*)*.*

Positions of at least one surgeon/assistance surgeon and at least one auxiliary operator, e.g. a nurse, within the operating room O during surgery are denoted with D an N, respectively.

Usually, four stitches are required for manually stitching a vessel of Ø 0.3 mm, said stitches being placed with an accuracy of +/-25% of an evenly-divided circumference.

**Fig. 2** provides a schematic illustration of a Ø 0.3 mm vessel and four stitch locations to perform the anastomosis.

Here, the distance between the stitching locations is indicated along with the required accuracy for successfully completing the stitching operation.

The same concept applies in case stitches are robotically applied during robot-assisted surgery, microsurgery, or super-microsurgery.

Vibrations at the instrument tip, e.g. due to floor vibrations, or other vibrations, e.g. connected to acceleration forces of the robot arm, may be prejudicial to precision and dexterity at the surgical location.

Therefore, it is important to prevent the occurrence of such undesired vibrations, which may hinder the required micrometer precision, dexterity and safety conditions.

Robotic systems for use in surgical, microsurgical or super-microsurgical procedures, provided with means directed at vibration reduction, are known in the art.

For instance, EP2015765322 discloses a patient-side wheeled cart for a teleoperated surgical system, said cart including a base, a column connected to the base, a boom connected to the column, a manipulator arm connected to the boom and configured to support a surgical instrument, and a vibration reduction member. In particular, the vibration reduction member may be configured for being moved between deployed and retracted positions relative to the base. The vibration reduction member may engage a ground surface in the deployed position and not be in contact with the ground surface in the retracted position.

A main drawback of these known solutions is that arm lengths and the center of gravity are often sub-optimal, this resulting in the occurrence of vibrations at the instrument tip and/or other undesired vibrations, e.g. due to acceleration forces of the robot arm, which may hinder precision and dexterity at the surgical target.

Therefore, there is the need for an enhanced solution capable of preventing the occurrence of such undesired vibrations when a surgical, microsurgical or super-microsurgical procedure is carried out.

In view of the above, it is an object of the present invention to provide a robotic system that allows an operator to reach a target location of a patient to perform a surgical, microsurgical or super-microsurgical procedure with improved precision and dexterity.

Also, the present invention aims at providing a robotic system capable of providing a stable foundation for one or more robot arms mounted therein, such that the performance of said robot arms can be optimized.

The above objects are obtained by the provision of a robotic system according to claim 1.

According to the invention, a robotic system for use in surgery, microsurgery or super-microsurgery comprises:
a base unit;
one or more robot arms, and
a positioning and suspension system, comprising:
   a beam assembly comprising:
   a first beam;
   a second beam, rotatably mounted atop the first beam, and
   a C-shaped beam, rotatably mounted atop the second beam, said C-shaped beam being configured to hold the one or more robot arms,
wherein the rotational axes of the first beam, the second beam and the C-shaped beam are parallel to each other, and
   a lift mechanism,
wherein the lift mechanism has a first end connected to the base unit and a second end rotatably connected to the first beam, said lift mechanism being configured to move in the upward or downward direction along its translational axis, parallel with respect to the rotational axes of the first beam, the second beam and the C-shaped beam, for adjusting a height of the beam assembly to compensate for the height of an operating table and the anatomy of a patient subject to surgery.

The present invention provides a robotic system for use in surgery, microsurgery or super-microsurgery.

The robotic system includes a base unit.

The robotic system further includes one or more robot arms.

Also, the robotic system comprises a positioning and suspension system.

The positioning and suspension system includes a beam assembly.

In particular, said beam assembly comprises:
a first beam;
a second beam, and
a C-shaped beam.

The second beam is rotatably mounted atop the first beam.

Similarly, the C-shaped beam is rotatably mounted atop the second beam.

The C-shaped beam is configured to hold the one or more robot arms.

The rotational axes of the first beam, the second beam and the C-shaped beam are parallel to each other.

The positioning and suspension system further comprises a lift mechanism.

In particular, the lift mechanism has a first end connected to the base unit and a second end rotatably connected to the first beam.

The lift mechanism is configured to move in the upward or downward direction along its translational axis, said translational axis of the lift mechanism being parallel with respect to the rotational axes of the first beam, the second beam and the C-shaped beam.

This vertical movement of the lift mechanism along its axis allows adjusting a height of the beam assembly to compensate for the height of an operating table and the anatomy of a patient subject to surgery.

The invention is based on the basic idea that, by the provision of a robotic system as described above, an operator, e.g. a surgeon, is enabled to perform a surgical, microsurgical or super-microsurgical procedure with improved precision and dexterity, and without the risk of mechanical interference between the beams the beam assembly. Also, the robotic system allows achieving an improved level of stability, thereby providing a stable foundation for the one or more robot arms.

Conveniently, the first beam and the second beam may have a length between 225 mm and 235 mm.

This enables first beam and the second beam to perform unlimited rotation without risk of mechanical interference.

The first beam and the second beam may have the same length.

This allows to improve the overall stability of the beam assembly, which is advantageous in order to enable the one or more robot arms to operate with high level of precision at the surgical target.

For example, the first beam and the second beam may have a length of 235 mm.

Nevertheless, a different length for the first and second beam, preferably falling within the above-recited range between 225 mm and 235 mm, is also possible according to the invention. Alternatively, the first and second beams may have different lengths.

The base unit may comprise a base frame.

The base unit may further comprise a plurality of wheels.

Advantageously, said plurality of wheels may be connected to the base frame.

The wheels are configured to rotate on the floor to allow for transport and positioning of the robotic system.

The base unit may further comprise a plurality of vertically-movable foot assemblies.

For instance, the base unit may comprise three vertically-movable foot assemblies.

This ensures a sufficient level of stability for the system.

Advantageously, the vertically-movable foot assemblies are also connected to the base frame.

Each of the vertically-movable foot assemblies is configured to switch between a retracted position for transport, and an extended position where said plurality of vertically-movable foot assemblies are in contact with the floor to stably maintain the robotic system in place to perform a surgical, microsurgical or super-microsurgical procedure.

Said plurality of vertically-movable foot assemblies is arranged externally with respect to said plurality of wheels.

Each of said vertically-movable foot assemblies may include a respective base portion comprising a dampening means.

The aforesaid arrangement of wheels and vertically-movable foot assemblies allows to easily and safely transport/position the robotic system, at the same time granting an enhanced level of stability of the system when a surgical, microsurgical or super-microsurgical operation is performed.

The dampening means allow to prevent the occurrence of dynamic harmonic motion excited by floor vibrations, which may affect the performance of the robot arms.

The lift mechanism may be connected to the base frame.

Preferably, said plurality of wheels includes four wheels.

In particular, said plurality of wheels may include a pair of rear swivel wheels and a pair of front wheels.

Since the front wheels are configured to rotate only in the forward/backward direction, the system can only move forwards and backwards, while it is not able to perform a side-by-side movement.

This enhances the overall stability of the system during transport, in particular on sloped surfaces.

When the robotic system is in a transport position, the position of the one or more robot arms entirely falls within the footprint of the robotic system, and said one or more robot arms are protected by the beam assembly.

This allows providing a sufficient protection against damage for these vulnerable components.

Also, during transportation of the robotic system, the operator has a good visibility on the robot arms as well as the front part of the robotic system.

Therefore, the robotic system can be moved/positioned with improved ease and safety.

The base unit may further comprise a first foot lever and a second foot lever.

The first foot lever and second foot lever are connected to a drive shaft of the base unit.

In particular, when the first foot lever is operated, the drive shaft determines a vertical movement of a foot shaft in the upward direction, thereby switching the vertically-movable foot assembly from the extended position toward the retracted position.

Similarly, when the second foot lever is operated, the drive shaft determines a vertical movement of the foot shaft in the downward direction, thereby switching the vertically-movable foot assembly from the retracted position toward the extended position.

Preferably, a pushing force that is exerted on the first foot lever or the second foot lever is transmitted to the drive shaft through a respective transmission rod.

Preferably, the foot shaft is arranged within a housing of each vertically-movable foot.

Therefore, the operator may conveniently switch the vertically-movable foot assemblies from the extended position to the retracted position, and *vice versa,* simply by acting on the respective foot lever with his/her foot.

The robotic system may further comprise a sensor, which is configured to detect whether the vertically-movable foot assemblies are in the retracted position or the extended position. Preferably, the sensor is an inductive sensor.

Advantageously, the inductive sensor is mounted on the base frame and is configured to detect a lever provided on the drive shaft, to thereby determine if the robotic system is standing on the vertically-movable foot assemblies or not.

The rotation of the drive shaft can be limited by the provision of a means, e.g. a lever, blocked by a stop block that is mounted on the base frame.

When the robotic system is standing on the vertically-movable foot assemblies, an excentre of each vertically-movable foot must be aligned with the drive shaft in the vertical direction, and thereby retained in place, e.g. by a stop block.

In this state, the excentre will create minimum/no moment on the drive shaft.

On the other hand, when the robotic system is in a transport position, i.e. it is standing on the rear and front wheels to allow for transport/positioning, a means, e.g. a gas spring, will keep the vertically-movable foot assemblies in the retracted position, in particular by pushing the lever against one of the stop blocks.

Accordingly, accidental displacement of the vertically-movable foot assemblies from their retracted position, which may cause interference during movement of the robotic system, can be largely prevented.

Advantageously, said plurality of vertically-movable foot assemblies may include three vertically-movable foot assemblies.

This allows achieving a sufficient level of stability for the robotic system when a surgical, microsurgical, or super-microsurgical operation is performed.

Based on the current regulations, the maximum operator force shall not exceed 150N.

Accordingly, a means, e.g. a gas spring, may be used to compensate some of the operating force.

The vertically movable foot assemblies can be provided with a means for generating pre-tension.

For instance, said means for generating pre-tension can be a disc spring.

This allows achieving sufficient stiffness in each vertically-movable foot assembly.

Advantageously, the dampening means of the vertically-movable foot assemblies can be made of rubber, preferably loaded in the shear direction.

Advantageously, said dampening means can be in the shape of a ring.

Preferably, said ring-shaped dampening means may be characterized by:
an outer diameter of 60 mm;
an inner diameter of 40 mm, and
a thickness of 2,5 mm.

As mentioned, this dampening means are appropriate in order to prevent the occurrence of dynamic harmonic motion excited by floor vibrations, which may affect the performance of the robot arms.

Advantageously, the C-shaped beam may include an indent configured to be grasped with the user's hand for manual positioning of the beam assembly.

Also, the C-shaped beam may include a plurality of control buttons.

Preferably, said plurality of control buttons *inter alia* includes a button which is operable for releasing brakes of the beam assembly.

Preferably, said plurality of control buttons further includes one or more buttons which are operable for determining the upward/downward vertical movement of the lift mechanism.

Accordingly, the operator is enabled to move/position the beam assembly and/or release the brakes and/or control the upward/downward movement of the lift mechanism with one hand.

For example, the operator can grab the indent to move the beam assembly, and use his/her thumb to press a button for releasing the brakes.

This significantly improves the operator's convenience during use.

Experiments revealed that an additional support to the base frame is desirable in order to obtain an appropriate level of stiffness for the robotic system.

To this end, the robotic system may be provided with a pair of rod-shaped supports for the lift mechanism.

Advantageously, one end of each support is connected to the lift mechanism, while the other end is connected to the base frame.

It is observed that, although the lift mechanism itself has a stiffness which may be considered high enough (i.e. 1 E6N/m), nevertheless the mounting interface at the base frame could be critical.

The problem is solved by the provision of said rod-shaped supports, which allow significantly improving the overall stiffness of the robotic system.

Advantageously, the base unit may be provided with a covering.

In particular, said covering may include:
- a pair of bumpers, mounted on the base unit;
- a pair of side covers;
- a front cover;
- a rear cover, and
- a connector cover.

For example, the components of the covering can be manufactured by implementing a 3D-printing process.

Also, a spray paint finish can be applied on the 3D-printed parts.

Alternatively, these components can be manufactured by vacuum molding.

Conveniently, the connector cover can be a hinged cover, in particular for hiding a detachable data cable connector and two fixed connections of power and protective earth cables.

Its function is to hide these components during normal use (both for cleaning and aesthetic purposes), at the same time allowing the user to easily reach and disconnect the data cable connector, when required.

The covering can be assembled to the base unit of the robotic system as follows:
- first, the bumpers are mounted to the base frame;
- then, the side covers are assembled, sliding in from the top. The side covers are mounted to the base frame as well;
- subsequently, the connector cover is assembled and secured, e.g. by screwing, to the base frame;
- then, the front cover is assembled by sliding it under the side covers, near the lift mechanism. A friction means can be used to keep the front cover in place. Also, in case said friction means are still not sufficient for the purpose, a Velcro^{®} means can be used;
- finally, the rear cover is assembled and secured, e.g. by screwing, near the swivel wheels.

Different colors can be used for one or more parts of the covering, to enhance the operator's convenience.

The first beam, the second beam and the C-shaped beam can be made of Aluminum.

Here, a powder coat finish can also be applied.

Also, the base frame can be made of a sheet metal sandwich panel.

This allows to obtain an appropriate level of flexibility, e.g. when compared to a solution using tubes welded together or large Aluminum milling parts, without prejudice to structural stability. The use of a metal sandwich panel also represents a cost-effective solution.

The base unit may further comprise a control cabinet, comprising a control means for the robotic system.

The base unit may further comprise a handlebar.

The handlebar is connected to the base frame.

The robotic system may further comprise a display means.

Accordingly, relevant information during a surgical, microsurgical or super-microsurgical operation can be readily made available to the operator through said display means.

The display means can be connected to the handlebar.

This positioning of the display means is convenient in order to allow the user to easily control relevant information displayed on the screen when a surgical, microsurgical or super-microsurgical operation is performed.

When the display means is mounted to the handlebar, cables of the display means can be routed inside the hollow tubes of the handlebar.

The handlebar may be provided with mechanical interfaces for the control cabinet and base frame.

Electronic components, such as a PCB and a PC, can be arranged within the control cabinet, along with a communication interface module of the lift mechanism.

Cabling is provided between these components and panel connectors provided on the outside of the control cabinet.

In order to provide sufficient strength to the handlebar, mechanical connections with the lift mechanism can be provided, so that push forces that are exerted on the handlebar can be neatly directed towards the wheels.

This prevents the handlebar from bending too much, which in turn prevents the handlebar from hitting the covers or giving the user the feeling of a badly designed product.

A different color, e.g. a darker color, can be used for components such as the handlebar, the foot levers and the indent on the C-shaped beam, to intuitively point out the interaction points to the operator.

Further details and advantages of the present invention shall now be disclosed in connection with the drawings, where:
**Fig. 1** is a schematic view of an operating room with a patient lying on an operating table, illustrating areas of the patient that need to be reached by a robotic system during a surgical, microsurgical or super-microsurgical procedure;
**Fig. 2** is a schematic view of a Ø 0.3 mm vessel, where four stitch locations are defined to perform an anastomosis;
**Fig. 3** is a perspective view of a robotic system according to an embodiment of the present invention;
**Fig. 4** is a view similar to that of **Fig. 3****,** but from a different perspective;
**Fig. 5** is a perspective view of a detail of the beam assembly of the robotic system according to the invention;
**Fig. 6** is a side view in vertical section of the beam assembly of **Fig. 5****;**
**Fig. 7** is a detail of the C-shaped beam, showing an indent for manual positioning of the beam assembly and a plurality of control buttons;
**Fig. 8** is a perspective view in cross-section of a detail of a shaft of the beam assembly;
**Fig. 9** is a perspective view of the robotic system of **Fig. 3****,** in a transport position;
**Fig. 10** is a view similar to that of **Fig. 9****,** but from a different perspective;
**Fig. 11** is a perspective view of a detail of the base unit of the robotic system of **Fig. 3****,** in larger scale;
**Fig. 12** is a perspective view of a detail of the control cabinet, the base frame and the lift mechanism of the robotic system of **Fig. 3****,** in larger scale;
**Fig. 13** is a view similar to that of **Fig. 12****,** but from a different perspective;
**Fig. 14** is a perspective view of a detail of the control cabinet and the handlebar of the robotic system of **Fig. 3****,** in larger scale;
**Fig. 15** is a perspective view in cross-section of a detail of one of the vertically-movable foot assemblies;
**Fig. 16** is a perspective view schematically illustrating movement transmission for switching the vertically-movable foot assemblies between the retracted position and the extended position;
**Fig. 17** is a detail view showing, inter alia, a sensor, in particular an inductive sensor, mounted on the base frame of the base unit, configured to detect whether the system is standing on the vertically-movable foot assemblies or not;
**Figs. 18-19** are detail front views in partial cross-section of one of the vertically-movable foot assemblies, in different states **(****Fig. 18****:** retracted position; **Fig. 19****:** extended position);
**Fig. 20** is a perspective, exploded view of the covering of the base unit;
**Fig. 21** is a diagram illustrating stiffness decomposition in the robotic system according to the invention, in the z-direction;
**Figs. 22-23** are diagrams illustrating Finite Element Method (FEM) calculations for the robotic system according to the invention in a first mode (rotation around the z-axis) and a second mode (rotation around the y-axis), respectively;
**Figs. 24-25** are diagrams reporting Finite Element Method (FEM) calculations for the base unit and the lift mechanism of the robotic system according to the invention, in particular to determine an optimal stiffness level;
**Fig. 26** is a diagram illustrating the range of motion of the beam assembly of the robotic system according to the invention in a given workspace. Here, the first beam and second beam both have length of 235 mm, and the center of the Z-lift mechanism is 350 mm from the edge of the operating table;
**Fig. 27** is a diagram illustrating the footprint layout of the robotic system according to the invention, in particular where a mathematical approach is applied for determining the arrangement of the vertically-movable foot assemblies and the wheels, based on the calculated center of gravity;
**Fig. 28** is a diagram illustrating the footprint layout of the robotic system according to the invention, as a result of the calculations of **Fig. 27****.**

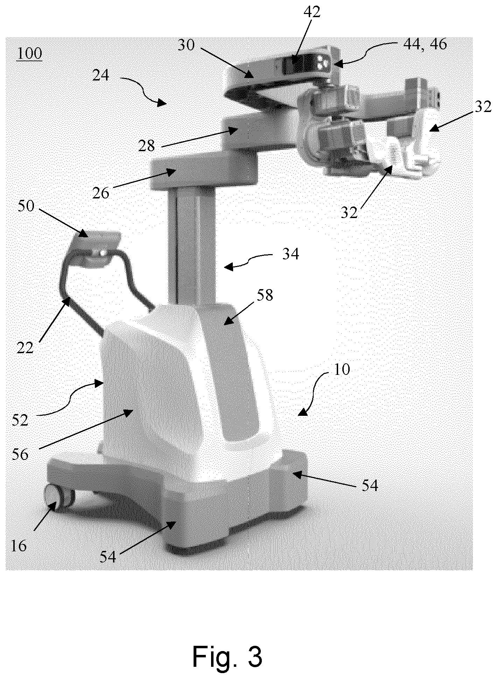

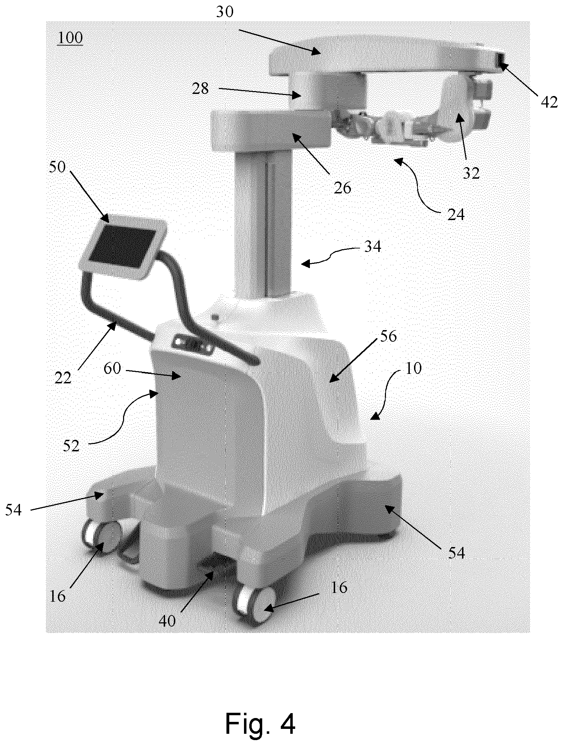

   **Figs. 3-4** show different perspective views of a robotic system 100 according to an embodiment of the present invention.

In particular, the robotic system 100 is configured for use in surgery, preferably microsurgery or super-microsurgery.

The robotic system 100 comprises a base unit 10.

Also, the robotic system 100 comprises one or more robot arms 32.

Still further, the robotic system 100 comprises a positioning and suspension system.

In particular, the positioning and suspension system comprises a beam assembly 24.

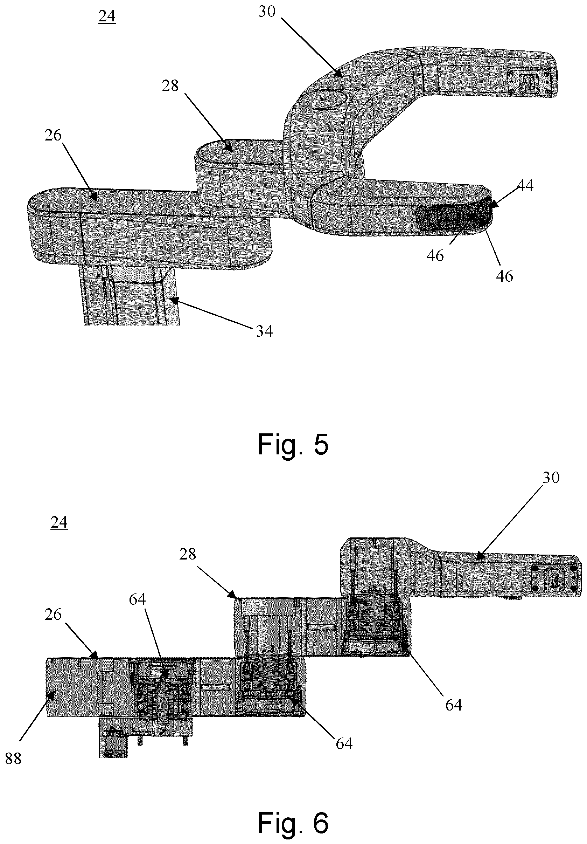

A detailed view of the beam assembly 24 of the robotic system 100 is provided in **Figs. 5-6**. In particular, in the present embodiment, the beam assembly 24 includes:
a first beam 26;
a second beam 28, and
a C-shaped beam 30.

The second beam 28 is rotatably mounted atop the first beam 26.

The C-shaped beam 30 is rotatably mounted atop the second beam 28.

The C-shaped beam 30 is configured to hold the one or more robot arms 32 **(****Figs. 3-4****)**.

The rotational axes of the first beam 26, the second beam 28 and the C-shaped beam 30 are parallel to each other **(****Figs. 5-6****)**.

The positioning and suspension system further comprises a lift mechanism 34.

The lift mechanism 34 has a first end connected to the base unit 10 and a second end rotatably connected to the first beam 26.

In the present embodiment, the first beam 26 and the second beam 28 have a length between 225 mm and 235 mm.

Accordingly, the first beam 26 and the second beam 28 are capable of performing unlimited rotation without risk of mechanical interference.

The first beam 26 and the second beam 28 may have the same length.

For example, the first beam 26 and the second beam 28 may both have a length of 235 mm.

However, a different length, preferably falling within the above-mentioned range between 225 mm and 235 mm is also possible.

Alternatively, the first and second beams 26, 28 may have different lengths, included in the above-specified range between 225 mm and 235 mm.

The lift mechanism 34 is configured to move in the upward or downward direction along its translational axis.

Accordingly, the height of the beam assembly can be adjusted to compensate for the height of an operating table (not shown) and the anatomy of a patient subject to surgery.

In particular, the translational axis of the lift mechanism 34 is perpendicular with respect to the rotational axes of the first beam 26, the second beam 28 and the C-shaped beam 30 **(****Figs. 3**-**4****).**

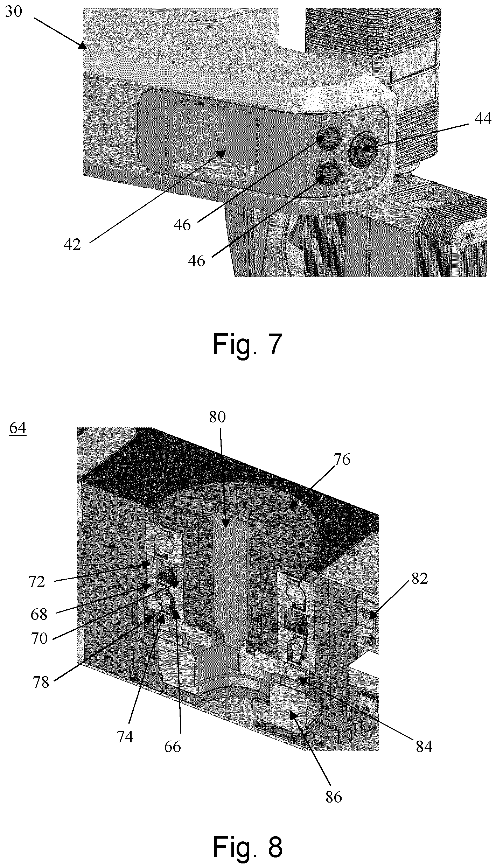

In the shown embodiment, the C-shaped beam 30 comprises an indent 42, configured to be grasped with the user's hand for manual positioning of the beam assembly 24 **(****Fig. 7****).**

Also, in the present embodiment, the C-shaped beam 30 further comprises a plurality of control buttons 44, 46.

In particular, in the present embodiment, the C-shaped beam 30 comprises a button 44 which is operable for releasing brakes of the beam assembly 24, and one or more buttons 46 which are operable, e.g., for determining the upward/downward vertical movement of the lift mechanism 34.

The buttons 44, 46 are arranged in a way that allows optimizing ease of use by the operator.

For instance, as shown in **Fig. 7****,** an upper button 46 and a lower button 46 can be provided, where the upper button 46 can be operated to determine a vertical movement of the lift mechanism 34 in the upward direction, while the lower button 46 can be operated to determine a vertical movement of the lift mechanism 34 in the downward direction.

The operator may move/position the beam assembly 24 by grabbing onto the indent 42 with his/her hand, and then release the brakes by acting on the button 44, e.g. with the thumb of the same hand.

Also, the upward/downward vertical movement of the lift mechanism 34 can be easily determined by acting on the buttons 46.

The button 44 for brakes release can be slightly larger than the buttons 46 for operating the lift mechanism 34, as shown in **Fig. 7****.**

The first beam 26 and the second beam 28 are provided with a bearing/brake assembly 64 **(****Fig. 6****).**

A detail of a bearing/brake assembly 64 is shown in **Fig. 8****.**

The bearing/brake assemblies 64 of the first and second beams 26, 28 have the same configuration **(****Fig. 6****).**

In order to achieve maximum stiffness, the bearing/brake assembly 64 is designed to be as compact as possible, with minimal parts.

The bearing/brake assembly 64 comprises inner rings 66 and outer rings 68.

A first bearing distance ring 70 is provided between the inner rings 66.

Similarly, a second bearing distance ring 72 is provided between the outer rings 68.

In order to reduce the play in the bearings and thereby increase stiffness, the second bearing distance ring 72 is +20 µm higher than the first bearing distance ring 70.

An inner bearing interface plate 74 is provided, pressing the bearing inner rings 66 against a flange 76.

As illustrated in **Fig. 8****,** the flange 76 of the bearing/brake assembly 64 of the first beam 26 provides an interface for connection with the second beam 28. Similarly, the flange 76 of the bearing/brake assembly 64 of the second beam 28 provides an interface for connection with the C-shaped beam 30.

An outer bearing interface plate 78 is provided to create pretension.

In particular, by providing pre-tension on the bearings, a high radial stiffness can be obtained.

A slip ring 80 is also provided, extending along the rotational axis of the bearing/brake assembly 64.

The slip ring 80 may be counted as a connection.

The bearing/brake assembly 64 further includes a PCB repeater 82.

This combination of slip rings and electronic repeaters are used in the linkage system, to ensure signal quality.

A brake rotor 84 and a brake stator 86 are provided in each bearing/brake assembly 64, as illustrated in **Fig. 8****.**

A counter mass 88 can be added at one end of the first robot 26 **(****Fig. 6****)** in order to improve stability of the beam assembly 24.

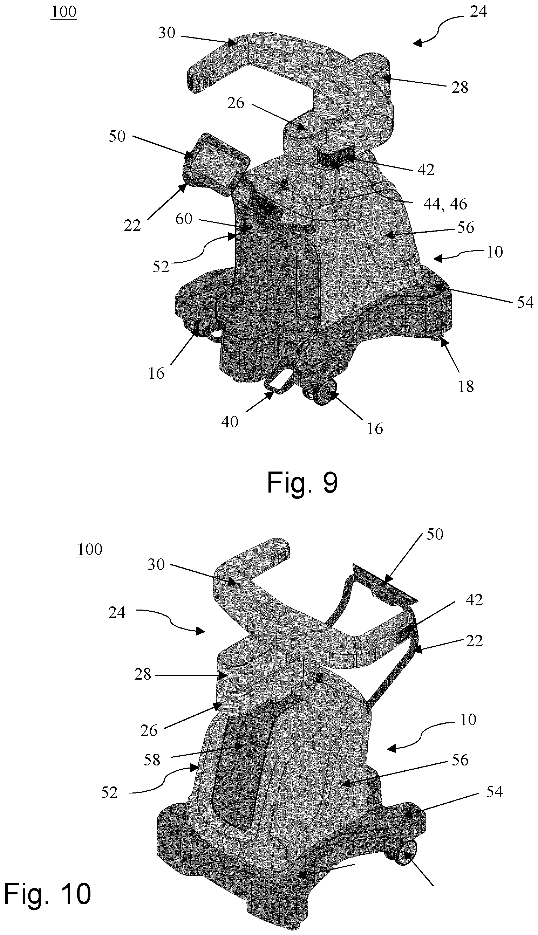

**Figs. 9-10** show the robotic system 100 in a transport position.

Here, the robot arms 32 (not shown in **Figs. 9-10**), which are vulnerable parts of the system 100, are entirely protected by the beam assembly 24.

In particular, in the transport position, the position of the robot arms 32 entirely falls within the footprint of the robotic system 100.

In the transport position **(****Figs. 9-10**) the operator has a good visibility on the one or more robot arms 32 and the front of the of the system 100.

In particular, the line of sight of the operator during transport is approximately underneath the C-shaped beam 30 toward the front of the robotic system 100.

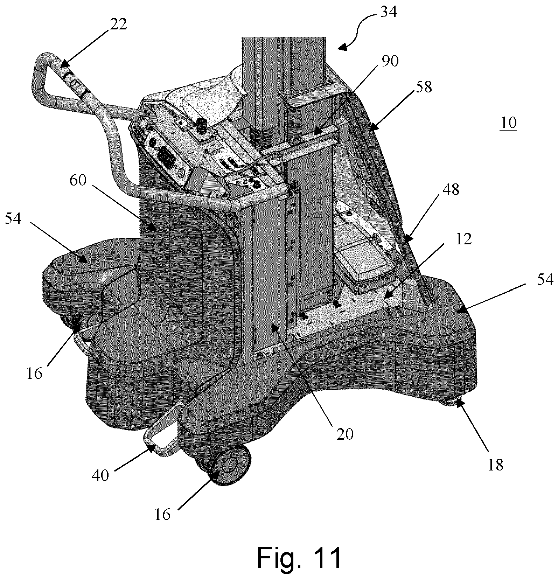

A detail of the base unit 10 of the system 100 is provided in **Fig. 11****.**

In the present embodiment, the base unit 10 comprises a base frame 12.

In this embodiment, the lift mechanism 34 is conveniently connected to the base frame 12.

The base unit 10 further comprises a plurality of wheels 14, 16, connected to the base frame 12.

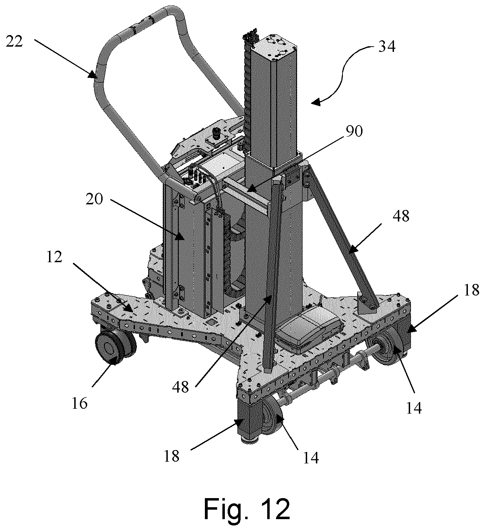

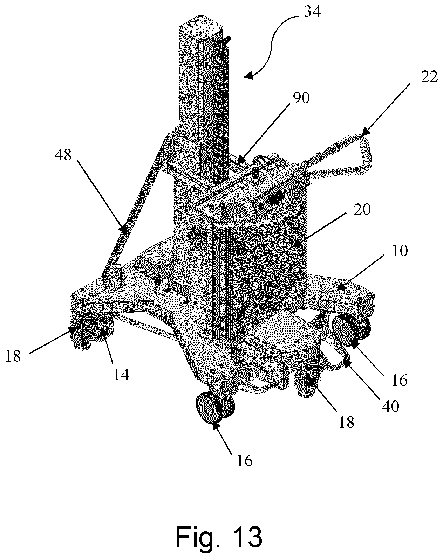

The wheels 14, 16 can be better seen in **Figs. 12-13**, providing views of the base unit 10 that are similar to that of **Fig. 11****,** but with omitted parts.

The wheels 14, 16 are configured to rotate on the floor to allow transport and positioning of the robotic system 100.

In particular, in the present embodiment, said plurality of wheels includes a pair of rear swivel wheels 16 and a pair of front wheels 14, which are configured to rotate only in the forward or backward direction.

Since the front wheels 14 are not able to swivel, but are only capable of rotating in the forward or backward direction, the robotic system 100 can only move in a forward or backward direction, and not side-to-side.

This allows to improve stability and ease during transport, in particular on sloped surfaces.

The base unit 10 further includes a plurality of vertically-movable foot assemblies 18, connected to the base frame 12 **(****Figs. 12-13****),** which will be described in further detail in the following.

In the present embodiment, the base unit 10 includes three foot assemblies 18.

This allows providing sufficient stability for the system 100.

The vertically-movable foot assemblies 18 are configured to switch between a retracted position for transport, and an extended position where said plurality of vertically-movable foot assemblies 18 are in contact with the floor to stably maintain the robotic system 100 in place during surgery, microsurgery or super-microsurgery.

The vertically-movable foot assemblies 18 are arranged externally with respect to said plurality of wheels 14, 16.

This allows obtaining enhanced stability when the robotic system 100 is standing on the vertically-movable foot assemblies 18.

Finite Element Method (FEM) calculations revealed that an additional support to the base frame 12 is appropriate in order to meet the overall stiffness requirements.

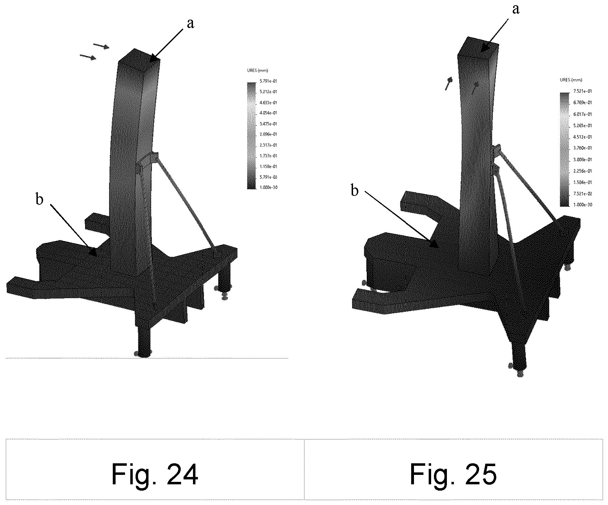

In particular, although the lift mechanism 34 has enough stiffness, nonetheless the mounting interface on the base frame 12 may still become critical (this aspect is explained in greater detail in the "experimental evidence" section below with reference to **Figs. 24-25****).**

To this end, a pair of rod-shaped supports 48 for the lift mechanism 34 are provided, as shown in **Figs. 11-13****.**

In particular, in this configuration, one end of each rod-shaped support 48 is connected to the lift mechanism 34, while the other end is connected to the base frame 12.

The rod-shaped supports 48 greatly increase the overall stiffness of the robotic system 100. In the present embodiment, the base unit 10 further comprises a control cabinet 20.

Also, the base unit 10 comprises a handlebar 22, connected to the base frame 12, enabling the operator to conveniently move/position the robotic system 100.

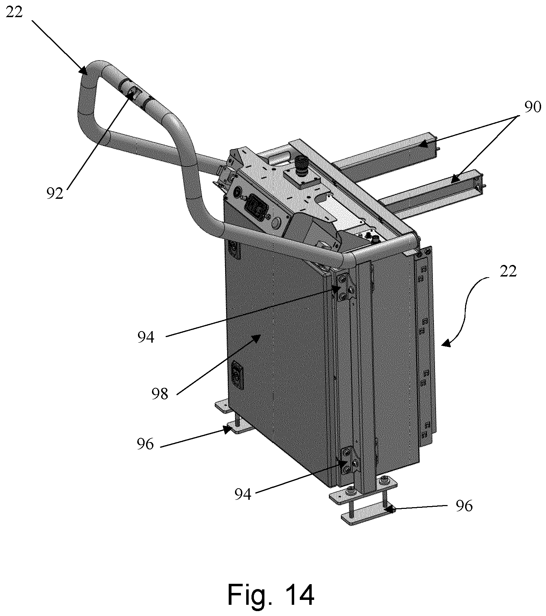

A detail view of the control cabinet 20 and handlebar 22 is provided in **Fig. 14****.**

In order to provide sufficient strength to the handlebar 22, mechanical connections 90, connecting the handlebar 22 to the lift mechanism 34, are provided **(****Figs. 12-13****).**

Accordingly, push forces that are exerted on the handlebar 22 can be directed towards the wheels 14, 16.

This prevents the handlebar 22 from bending too much, which in turn prevents the handlebar 22 from hitting a covering 52 (described in greater detail in the following) or giving the user the feeling of a badly designed product.

In the present embodiment, the robotic system 100 further comprises a display means 50.

Relevant information concerning a surgical, microsurgical or super-microsurgical operation that is being carried out on a patient can be displayed on the display means 50 to be readily accessible for the operator.

In the present embodiment, the display means 50 is connected to the handlebar 22 **(****Figs. 3-4** and **9).**

This positioning of the display means 50 is convenient to allow the operator to easily access the displayed information.

Electronic components, e.g. a PCB and a PC, can be arranged within the control cabinet 20 along with a communication interface module of the lift mechanism 34.

Cabling is provided between these components and panel connectors provided on the outside of the control cabinet 20.

In the present embodiment, the handlebar 22 is provided with an interface 92 for the display means 50 **(****Fig. 14****).**

Cables of the display means 50 are conveniently routed inside hollow tubes of the handlebar 22.

Also, the handlebar 22 has mechanical interfaces 94, 96 for the control cabinet 20 and the base frame 12, respectively **(****Fig. 14****).**

The control cabinet 20 is provided with a door 98, allowing easy access to the electronic components arranged therein, e.g. for maintenance.

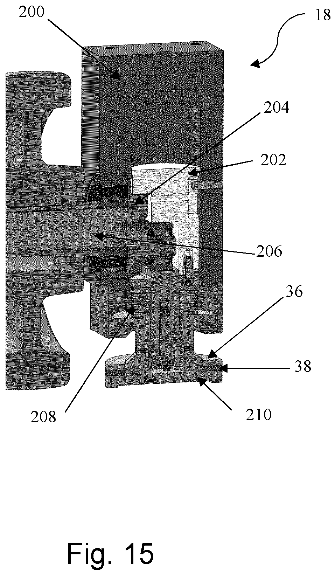

The vertically-movable foot assemblies 18 will now be described in greater detail referring to **Figs. 15-19****.**

**Fig. 15** provides a cross-section view of one of the vertically-movable foot assemblies 18.

The vertically-movable foot assemblies 18 all have the same structure.

Each of the plurality of vertically-movable foot assemblies 18 includes a base portion 36 comprising a dampening means 38, to suppress dominant vibration modes.

In the present embodiment, said dampening means 38 is made of rubber, preferably preloaded in the shear direction.

In the present embodiment, said dampening means 38 is in the shape of a ring **(****Fig. 15****).**

In particular, said ring-shaped dampening means 38 are characterized by:
an outer diameter of 60 mm;
an inner diameter of 40 mm, and
a thickness of 2,5 mm.

As shown in **Fig. 15****,** each vertically-movable foot assembly 18 includes a foot housing 200, enclosing and protecting the mechanical components of each vertically-movable foot assembly 18.

Each foot assembly 18 further comprises a foot shaft 202 and an excentre 204, the excentre 204 being connected to a drive shaft 206 which drives a movement of the vertically-movable foot assembly 18 between the retracted position and the extended position.

A disc spring 208 is provided in each foot assembly 18, allowing the foot assembly 18 to switch from the retracted position to the extended position, and vice versa.

The disc spring 208 provides pre-tensioning.

In particular, pre-tensioning generated by means of the disc spring 208 is tuned so as to provide enough stiffness in the foot assembly 18.

Finally, a foot plate 210 is provided at the bottom of the base portion 36.

The foot plate 210 is configured to be in contact with the floor of the operating room, and cooperates to reliably maintain the robotic system 100 in place when standing on the vertically-movable foot assemblies 18.

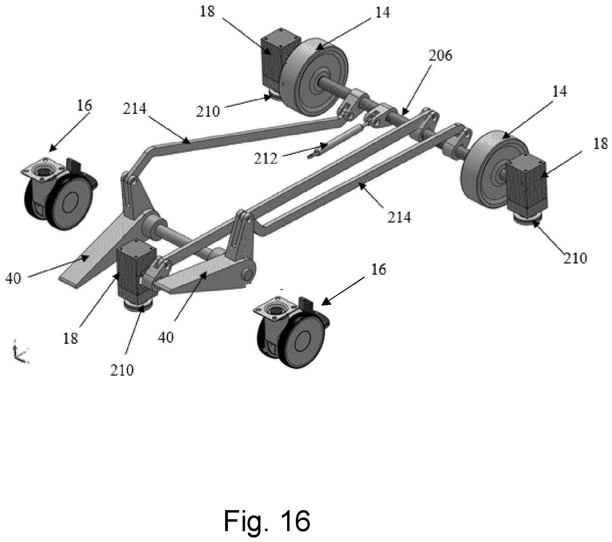

**Fig. 16** schematically illustrates the movement transmission mechanism for switching the vertically-movable foot assemblies 18 between the retracted position and the extended position.

The base unit 10 further comprises a first foot lever 40 and a second foot lever 40, connected to the drive shaft 206.

When the first foot lever 40 is operated, the drive shaft 206 determines a vertical movement of a foot shaft 202 in the upward direction, thereby switching the vertically-movable foot assembly 18 from the extended position toward the retracted position.

Similarly, when the second foot lever 40 is operated, the drive shaft 206 determines a vertical movement of the foot shaft 202 in the downward direction, thereby switching the vertically-movable foot from the retracted position toward the extended position.

The foot shaft 202 is arranged within the foot housing 200.

As shown in **Fig. 16****,** a pushing force that is exerted on the first foot lever 40 or the second foot lever 40 is transmitted to the drive shaft 206 through a respective transmission rod 214.

Since, according to the current regulations, an exerted operating force may not exceed 150N, a means, e.g. a gas spring 212, is provided to compensate some of the operating force.

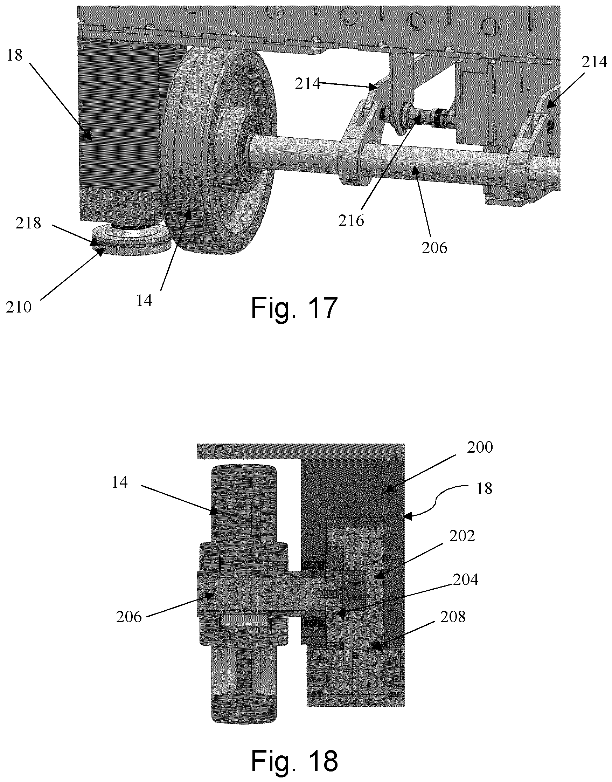

The robotic system 100 further comprises a sensor 216, preferably an inductive sensor 216, which is configured to detect whether the vertically-movable foot assemblies 18 are in the retracted position or the extended position **(****Fig. 17****).**

Preferably, the inductive sensor 216 is mounted on the base frame 12, as illustrated in **Fig. 17****.**

In particular, the inductive sensor 216 may be configured to detect a lever mounted either on the drive shaft 206 or the vertically-movable foot assemblies 18, to thereby determine if the robotic system 100 is standing on it the vertically-movable foot assemblies 18 or not.

The rotation of the drive shaft 206 can be limited by the provision of a lever blocked by a stop block mounted on the base frame 12.

When the robotic system 100 is standing on the vertically-movable foot assemblies 18, the excentre 204 must be aligned with drive shaft 206 in the vertical direction, and thereby retained in place, e.g. by a stop block.

In this state, the the excentre 204 will create minimum/no moment on the drive shaft 206.

On the other hand, when the robotic system 100 is in the transport position, standing on the wheels 14, 16, the gas spring 212 will keep the vertically-movable foot assemblies 18 in the retracted position by pushing the lever against one of the stop blocks provided on the base frame 12.

**Fig. 18** shows a detail of one of the vertically-movable foot assemblies (18) in the retracted position.

Here, a single foot assembly 18 is illustrated in the figure for the sake of simplicity.

In this condition, the robotic system 100 is standing on the wheels 14, 16, for transport.

A single wheel 14 is illustrated in the figure, said wheel 14 being in contact with the floor.

In the present embodiment, where the base unit includes three foot assemblies 18, a pre-tension of the disc springs 208 is 130% of 1/3 of the robotic system mass.

The force exerted on the excentre 204 corresponds to the pre-tension of the disc springs 208.

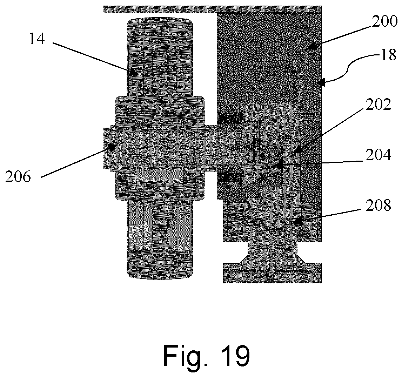

**Fig. 19** shows a detail of one of the vertically-movable foot assemblies 18 in the extended position.

Similar as above, a single foot assembly 18 is illustrated in the figure for the sake of simplicity.

Same as above, in the configuration according to the present embodiment, the pre-tension of the disc springs 208 is 130% of 1/3 of the robotic system mass.

Also, the force exerted on the excentre 204 corresponds to the pre-tension of the disc springs 208.

The operating force has to overcome the 130% of the total system mass.

The operating force is reduced by a supporting gas spring 212 mounted in the actuation system, which compensates at least 65% of the total system mass.

When the foot assemblies 18 are in an extended position (as shown in **Fig. 19****),** the primary contact area of each foot assembly 18 with the floor is the foot plate 210.

Preferably, the foot plate 210 comprising a rubber disc 218 to ensure mechanical decoupling and damping of the system 100.

In the present embodiment, the base unit 10 further includes a covering 52.

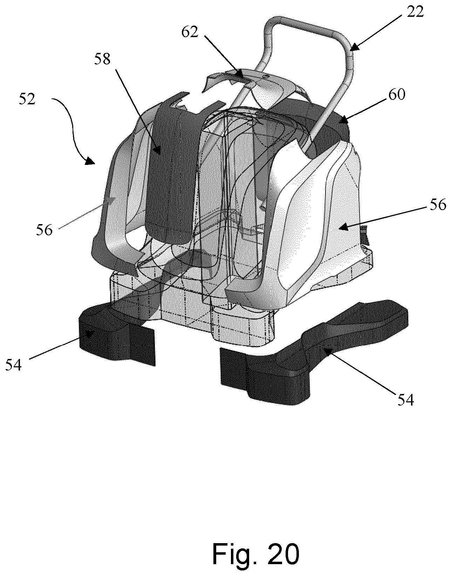

In particular, as shown in **Fig. 20****,** the covering 52 comprises:
- a pair of bumpers 54, mounted on the base unit 10;
- a pair of side covers 56;
- a front cover 58;
- a rear cover 60, and
- a connector cover 62.

The components 54, 56, 58, 60, 62 of the covering 52 can be manufactured by implementing a 3D-printing process.

Also, a spray paint finish can be applied on the 3D-printed parts.

Alternatively, these components can be manufactured by vacuum molding.

Different colors can be used for one or more parts of the covering 52, to enhance the operator's convenience.

The covering 52 can be assembled to the base unit 10 as follows:
- first, the bumpers 54 are mounted to the base frame 12;
- then, the side covers 56 are assembled, sliding in from the top. The side covers 56 are mounted to the base frame 12 as well;
- subsequently, the connector cover 62 is assembled and secured, e.g. by screwing, to the base frame 12;
- then, the front cover 58 is assembled by sliding it under the side covers 56, near the lift mechanism 34. A friction means (not shown) can be used to keep the front cover 58 in place. Also, in case said friction means are still not sufficient for the purpose, a Velcro^{®} means (not shown) can be used;
- finally, the rear cover 60 is assembled and secured, e.g. by screwing, near the swivel wheels 16.

In the present embodiment, the first beam 26, the second beam 28 and the C-shaped beam 30 are made of Aluminum.

Advantageously, a powder coat finish can be applied.

A different color, e.g. a darker color, can be used for components such as the handlebar 22, the foot levers 40 and the indent 42 on the C-shaped beam 30, to intuitively point out the interactions point to the operator.

Also, in the present embodiment, the base frame 12 can be made of a sheet metal sandwich panel.

This allows to obtain an appropriate level of flexibility, e.g. when compared to a solution using tubes welded together or large Aluminum milling parts, without prejudice to structural stability. The use of a metal sandwich panel also represents a cost-effective solution.

### EXPERIMENTAL EVIDENCE

### Required accuracy per vessel diameter and error budget

The required accuracy per vessel diameter is illustrated in **Table 1.** Here, three use cases were determined for different vessel diameters. In particular:
- use case 1: vessel diameter 1 - 2 mm.
- use case 2: vessel diameter 0.5 - 1 mm.
- use case 3: vessel diameter 0.3 - 0.5 mm

### Stiffness decomposition

A FEM analysis for system stiffness has been carried out considering the three different cases described above **(Table 1).**

For the purpose of this analysis, an error budget was defined for each of the above-mentioned use cases 1 to 3.

Also, twelve separate systems components, identified as the major contributors to system stiffness, have been considered.

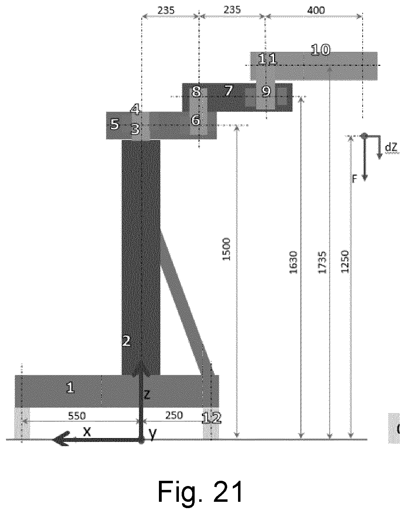

In particular, said twelve components include **(****Fig. 21****):**
- Base frame **(1),**
- Lift mechanism **(2),**
- First bearing **(3),**
- First brake **(4),**
- First link **(5),**
- Second bearing **(6),**
- Second link **(7),**
- Second brake **(8),**
- Third bearing **(9),**
- C-shaped beam **(10),**
- Third brake **(11),** and
- Vertically-movable foot assemblies **(12).**

Total stiffness was calculated from the virtual consideration of the individual stiffnesses of the aforementioned twelve components.

For the calculation of the individual stiffnesses, the point of interest, i.e. the tooltip of the robot arm, was used as the starting point and reference length.

The stiffness of the robotic system results from the respective stiffness shapes.

The stiffness of the robotic system describes the resistance to deformation.

In particular, the higher the stiffness of a component, the more force must be applied for it to deform.

Conversely, the higher the stiffness of the individual elements of the robotic system, the more force the individual component can absorb.

The axial stiffness is the product of the elastic modulus and the cross-section of the component divided by the length or distance to the point of interest, i.e. the tooltip of the robot arm.

The individual stiffnesses of the twelve components can be added together to give the total stiffness of the system.

Preferably, the maximum force is applied at the point of interest.

The total stiffness and the individual stiffnesses are considered virtually in the z-direction **(****Fig. 21****).**

The component with the highest extensional stiffness is the base frame **(1).**

In particular, this component must absorb the totality of all forces of the robotic system.

Thus, the base frame preferably has the highest stiffness among the system components.

The lowest stiffness can be allowed in the C-shaped beam **(10).**

### Expected dynamics

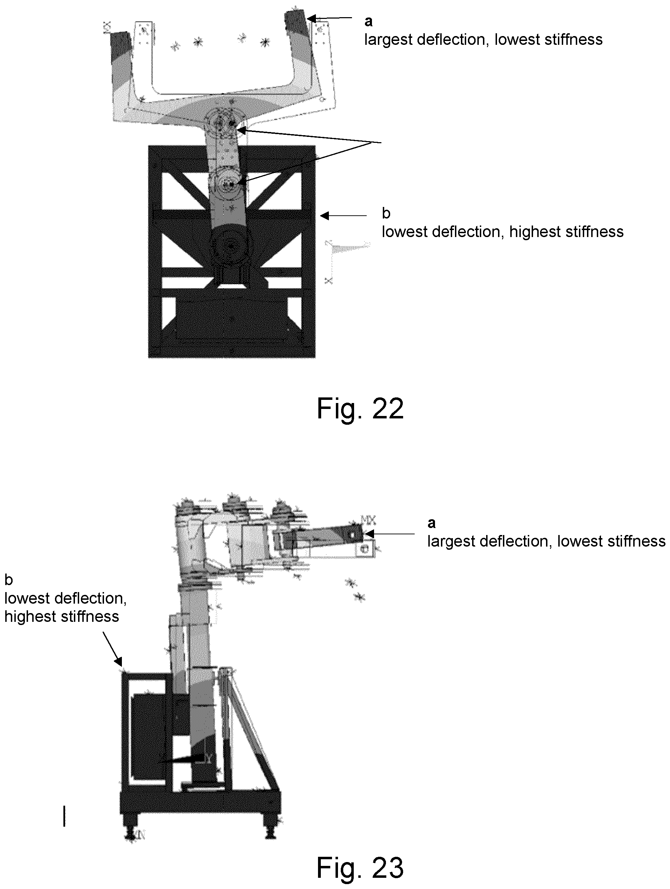

**Fig. 22** illustrates the expected dynamics for the robotic system of the invention in a first mode (rotation around the z-axis).

**Fig. 23** illustrates the expected dynamics for the robotic system of the invention in a second mode (rotation around the y-axis).

Here, areas denoted with (a) represent areas characterized by the largest deflection and the lower stiffness, while areas denoted with (b) represent areas characterized by the lowest deflection and the highest stiffness.

A vibratory system consisting of springs and masses has characteristic natural modes with associated natural frequencies, therefore the natural modes and frequencies of the robotic system were calculated.

By changing the masses or spring stiffnesses, the natural frequencies can be affected.

The number of natural modes depends on the number of degrees of freedom of the system. In principle, the natural frequencies of the robotic system should be as high as possible.

For the mode angular rotation around the z-axis, only the stiffness of the first and second beams and the C-shaped beam has to be considered.

It can be assumed that the axis of rotation in the z-direction is on the z-lift.

Also, it can be advantageous to determine the moment of inertia of the robotic system for z-axis rotation

For this purpose, the mass of the first beam, the second beam and the C-shaped beam, as well as their distance from the axis of rotation, are taken into account **(****Fig. 22****).**

It can be favorable if the rotation axis is also considered in the y-direction **(****Fig. 23****).**

To estimate the natural frequency, the above-mentioned virtually calculated stiffness at the point of interest, i.e. the tooltip of the robot arm, is used.

For the rotation around the y-axis, all aforementioned twelve components **(****Figs. 21****)** are considered.

Also, it can be advantageous to determine the moment of inertia of the robotic system for y-axis rotation, as in the z-direction.

The natural frequency in the y-direction is almost twice as high as the natural frequency of the robotic system in the z-direction.

### FEM analysis of the lift mechanism and the base frame

FEM analysis of the lift mechanism and the base frame was performed.

The results of this analysis are schematically illustrated in **Figs. 24-25****.**

In particular, the results revealed that it might be advantageous that the base frame is provided with additional support(s), e.g. rod shaped-supports as illustrated in **Figs. 24-25****,** in order to meet the overall stiffness requirements for the robotic system.

Although the lift mechanism has a sufficiently high stiffness (1 E6N/m), nevertheless the attachment interface at the base frame may become critical **(****Figs. 24-25****).**

Therefore, it is favorable to provide additional support(s), e.g. rod-shaped supports, as shown in **Figs. 24-25****.**

It is noted that the provision of the additional support(s) significantly increases the overall stiffness of the system.

The deflection (in mm) is greatest in the upper part of the lift mechanism.

On the other hand, the smallest deflection occurs at the lower end of the lift mechanism **(****Figs. 24-25****).**

In particular, deflection is the greatest in the areas denoted with **(a)** in **Figs. 24-25** and the smallest in the areas denoted with **(b)** in **Figs. 24-25****.**

### Range of motion of the beam assembly in a given surgical workspace

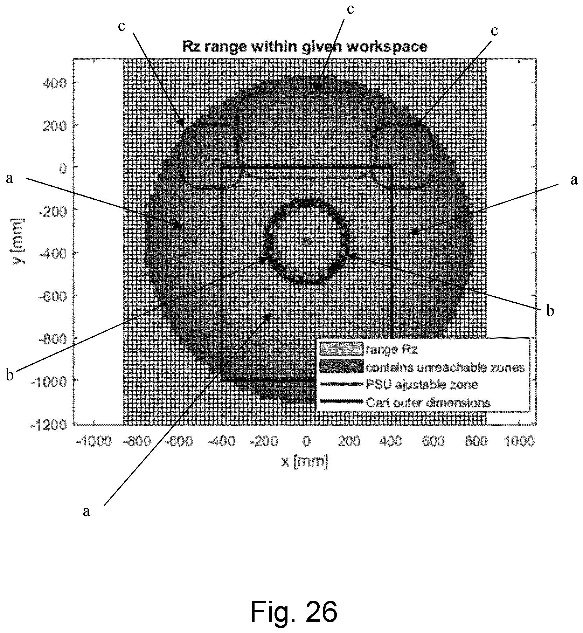

**Fig. 26** illustrates the range of motion of the beam assembly of the robotic system according to the invention, in a given surgical workspace.

Here, the first beam and the second beam both have a length of 235 mm.

The center of the lift mechanism is set at 350 mm from the edge of the operating table.

The areas denoted with **(a)** in **Fig. 26** represent the range of motion of the beam assembly in an optimized state.

In particular, this means that the beam assembly allows reaching the entire area, and is also able to perform the required rotation around the z-axis (Rz).

The points denoted with **(b)** in **Fig. 26** represent points that can be reached by the beam assembly, but where the beam assembly is not able to perform the required rotation around the z-axis (Rz).

The areas defined with lines **(c)** in the upper part of **Fig. 26** represent the outermost areas of the surgical workspace, which are most difficult to reach during a surgical, microsurgical or super-microsurgical procedure.

In the robotic system of the invention, the only constraint is that the robot arms can hit the second beam in certain positions.

In particular, this limit has been estimated as +-110 degrees, measured from the C-shaped beam to the second beam.

### Footprint layout (arrangement of vertically-movable foot assemblies and wheels)

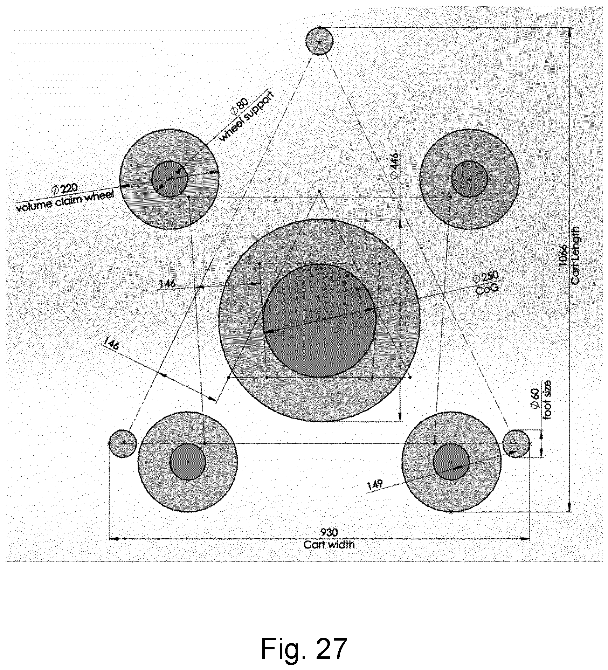

A preferred footprint layout for the robotic system of the invention is illustrated in detail in **Fig. 27****.**

In this preferred arrangement, the system includes 3 vertically-movable foot assemblies and 4 wheels, attached to the base frame.

In order to determine the optimal arrangement for the vertically-movable foot assemblies and wheels, a mathematical approach has been adopted, which is based on the calculation of the center of gravity of the robotic system.

In particular, the center of gravity should be within the region defined by the support points **(****Fig. 27****).**

The performed calculations further revealed that adding weight to the bottom of the system (i.e. at base frame level), as well as the addition of a counter-mass, could be appropriate for compliance with the current regulations, at the same time optimizing the overall weight of the robotic system.

### Footprint layout (stability I center of gravity)

The large triangle in **Fig. 27** schematically represents the region of stability of the robotic system without applied push force.

On the other hand, the small triangle in **Fig. 27** schematically represents the region of stability of the robotic system when an operator is pushing/pulling the robotic system with 150N at a height of 1500 mm from any direction.

Still further, the small circle in **Fig. 27**schematically represents the center of gravity of the robotic system, including the robot arms.

In particular, said small circle must be included within the boundaries defined by the small triangle (conditions: inclination of 0° and pushing/pulling with 150N).

On the other hand, the large circle in **Fig. 27** schematically represents the center of gravity of the robotic system, including the robot arms, when standing on a floor with a slope of 5° in any direction.

In particular, said large circle must be included within the boundaries defined by the large triangle.

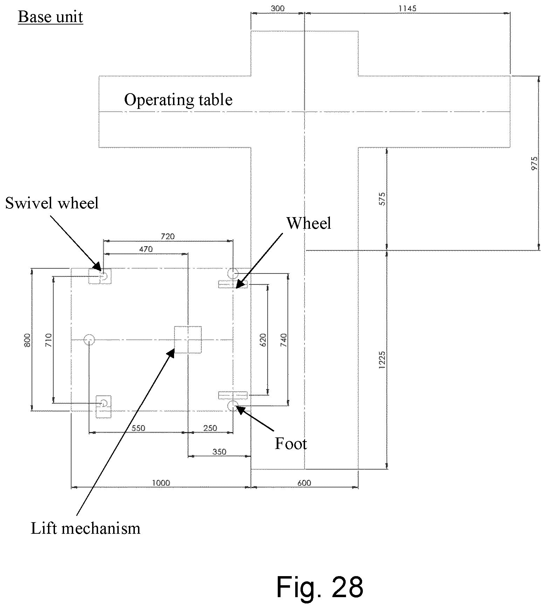

An optimized footprint layout for the robotic system in an operating position at the bedside, is schematically represented in **Fig. 28****.**

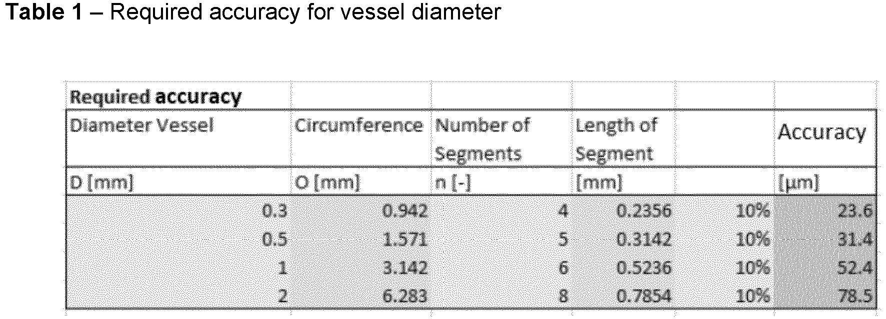

### Reference list

- 100: Robotic system

- 10: Base unit
- 12: Base frame
- 14: Wheel
- 16: Swivel wheel
- 18: Vertically-movable foot assembly
- 20: Control cabinet
- 22: Handlebar
- 24: Beam assembly
- 26: First beam
- 28: Second beam
- 30: C-shaped beam
- 32: Robot arm(s)
- 34: Lift mechanism
- 36: Base portion (vertically-movable foot)
- 38: Dampening means
- 40: Foot lever
- 42: Indent (C-shaped beam)
- 44, 46: Control button(s)
- 48: Rod-shaped support(s)
- 50: Display means
- 52: Covering (base unit)
- 54: Bumpers (covering)
- 56: Side covers (covering)
- 58: Front cover (covering)
- 60: Rear cover (covering)
- 62: Connector cover (covering)
- 64: Bearing/brake assembly (beam assembly)
- 66: Inner ring(s)
- 68: Outer ring(s)
- 70: First bearing distance ring
- 72: Second bearing distance ring
- 74: Inner bearing interface plate
- 76: Flange
- 78: Outer bearing interface plate
- 80: Slip ring
- 82: PCB repeater
- 84: Brake rotor
- 86: Brake stator
- 88: Counter mass
- 90: Mechanical connections (handlebar)
- 92: Interface for the display means
- 94: Interface for the control cabinet
- 96: Interface for the base frame
- 98: Door of the control cabinet
- 200: Foot housing
- 202: Foot shaft
- 204: Excentre
- 206: Drive shaft
- 208: Disc spring
- 210: Foot plate
- 212: Gas spring
- 214: Transmission rod
- 216: Sensor, inductive sensor
- 218: Rubber disc

- O: Operating room
- P: Patient
- T: Operating table
- S: Robotic system
- A: Surgical location(s)
- D: Position of a surgeon/assistant surgeon in the operating room
- N: Position of an auxiliary operator in the operating room

## Claims

1. A robotic system (100) for use in surgery, microsurgery or super-microsurgery, said robotic system (100) comprising:
a base unit (10);
one or more robot arms (32), and
a positioning and suspension system, comprising:
a beam assembly (24) comprising:
a first beam (26);
a second beam (28), rotatably mounted atop the first beam (26), and
a C-shaped beam (30), rotatably mounted atop the second beam (28), said C-shaped beam (30) being configured to hold the one or more robot arms (32),
wherein the rotational axes of the first beam (26), the second beam (28) and the C-shaped beam (30) are parallel to each other, and
a lift mechanism (34),
wherein the lift mechanism (34) has a first end connected to the base unit (10) and a second end rotatably connected to the first beam (26), said lift mechanism (34) being configured to move in the upward or downward direction along its translational axis,
parallel with respect to the rotational axes of the first beam (26), the second beam (28) and the C-shaped beam (30), for adjusting a height of the beam assembly (24) to compensate for the height of an operating table and the anatomy of a patient subject to surgery.

2. The robotic system (100) according to claim 1,
**characterized in that**
the base unit (10) comprises:
- a base frame (12);
- a plurality of wheels (14, 16), connected to the base frame (12), said plurality of wheels (14, 16) being configured to rotate on the floor to allow for transport and positioning of the robotic system (100);
- a plurality of vertically-movable foot assemblies (18), connected to said base frame (12), each configured to switch between a retracted position for transport and an extended position where said plurality of vertically-movable foot assemblies (18) are in contact with the floor to stably maintain the robotic system (100) in a determined position to perform surgery, microsurgery or super-microsurgery,
wherein said plurality of vertically-movable foot assemblies (18) is arranged externally with respect to said plurality of wheels (14, 16), and
wherein each of said plurality of vertically-movable foot assemblies (18) includes a base portion (36) comprising a dampening means (38).

3. The robotic system (100) according to claim 1 or 2,
**characterized in that**
the first beam (26) and the second beam (28) each have a length between 225 mm and 235 mm.

4. The robotic system (100) according to claim 3,
**characterized in that**
the first beam (26) and the second beam (28) have the same length.

5. The robotic system (100) according to any of claims 2 to 4,
**characterized in that**
the lift mechanism (34) is connected to the base frame (12).

6. The robotic system (100) according to any one of claims 2 to 5,
**characterized in that**
said plurality of wheels (14, 16) includes:
a pair of rear swivel wheels (16), and
a pair of front wheels (14), configured to rotate only in the forward or backward direction.

7. The robotic system (100) according to any one of claims 2 to 6,
**characterized in that**
the base unit (10) further comprises a first foot lever (40) and a second foot lever (40), connected to a drive shaft (206) of the base unit (10),
wherein, when the first foot lever (40) is operated, the drive shaft (206) determines a vertical movement of a foot shaft (202) in the upward direction, thereby switching the vertically-movable foot assembly (18) from the extended position toward the retracted position, while, when the second foot lever (40) is operated, the drive shaft (206) determines a vertical movement of the foot shaft (202) in the downward direction, thereby switching the vertically-movable foot assemblies (18) from the retracted position toward the extended position,
preferably wherein a pushing force exerted on the first foot lever or the second foot lever (40) is transmitted to the drive shaft (206) through a respective transmission rod (216).

8. The robotic system (100) according to any one of claims 2 to 7,
**characterized in that**
the robotic system (100) further comprises a sensor (216), preferably an inductive sensor (216), which is mounted on the base frame (12) and is configured to detect whether said plurality of vertically-movable foot assemblies (18) is in the retracted position or the extended position.

9. The robotic system (100) according to any one of claims 2 to 8,
**characterized in that**
said plurality of vertically-movable foot assemblies (18) includes three vertically-movable foot assemblies (18).

10. The robotic system (100) according to any one of claims 2 to 9,
**characterized in that**
said dampening means (38) is made of rubber, preferably loaded in the shear direction, preferably wherein said dampening means (38) is in the shape of a ring,
preferably wherein said ring has:
an outer diameter of 60 mm;
an inner diameter of 40 mm, and
a thickness of 2,5 mm.

11. The robotic system (100) according to any one of the preceding claims,
**characterized in that**
said C-shaped beam (30) includes:
an indent (42) configured to be grasped with the user's hand for manual positioning of the beam assembly (24), and
a plurality of control buttons (44, 46),
preferably wherein said plurality of control buttons (44, 46) includes:
a button (44) which is operable for releasing brakes of the beam assembly (24), and
one or more buttons (46) which are operable for determining the upward or downward movement of the lift mechanism (34).

12. The robotic system (100) according to any one of claims 2 to 11,
**characterized in that**
the robotic system (100) further comprises a pair of rod-shaped supports (48) for the lift mechanism (34), wherein one end of each support (48) is connected to the lift mechanism (34), while the other end is connected to the base frame (12).

13. The robotic system (100) according to any one of the preceding claims,
**characterized in that**
the base unit (10) further includes a covering (52), comprising:
- a pair of bumpers (54), mounted on the base unit (10);
- a pair of side covers (56);
- a front cover (58);
- a rear cover (60), and
- a connector cover (62).

14. The robotic system (100) according to any one of the preceding claims,
**characterized in that**
the first beam (26), the second beam (28), and the C-shaped beam (30) are made of Aluminum.

15. The robotic system (100) according to any one of claims 2 to 14,
**characterized in that**
the base frame (12) is made of a sheet metal sandwich panel.

16. The robotic system (100) according any one of the preceding claims,
**characterized in that**
the base unit (10) further comprises:
- a control cabinet (20) comprising a control means for the robotic system (100), and
- a handlebar (22), connected to the base frame (12),
preferably wherein the robotic system (100) further comprises a display means (50),
preferably wherein the display means (50) are connected to the handlebar (22).
